# EUROPÄISCHE PATENTSCHRIFT

(11) **EP 0 778 013 B1**
(45) Veröffentlichungstag und Bekanntmachung des Hinweises auf die Patenterteilung: **23.05.2001**
(21) Anmeldenummer: 96114830.1
(22) Anmeldetag: 16.09.1996
(51) Int. Cl.: A61F 2/52

(54) **Brustprothese**
Mammary prosthesis
Prothèse mammaire

(30) Priorität: 05.12.1995 DE 29519283 U
(43) Veröffentlichungstag der Anmeldung: 11.06.1997
(73) Patentinhaber: AMOENA Medizin-Orthopädie-Technik GmbH, D-83064 Raubling (DE)
(72) Erfinder: Wild, Helmut, D-83115 Neubeuern (DE)
(74) Vertreter: Laufhütte, Dieter, Dr.-Ing.

(56) Entgegenhaltungen:
- EP-A- 0 320 590
- DE-A- 4 413 076
- DE-U- 9 315 935

## Beschreibung

Die Erfindung betrifft eine Brustprothese, bestehend aus einem schalenförmigen Körper aus einem weich-elastisch eingestellten Kunststoff, vorzugsweise einer additionsvernetzten Zwei-Komponenten-Silikon-Kautschuk-Masse, der in Kunststoffolien eingeschweißt ist und so eine erste Kammer bildet, und ein aus einer der Trägerin während des Tragens zugewandten und sich an die erste Kammer anschließenden mit einem anderen Füllmaterial gefüllten zweiten Kammer.

Brustprothesen, die aus einem schalenförmigen Körper aus einem weich-elastisch eingestellten additionsvernetzten Zwei-Komponenten-Silikon-Kautschuk bestehen, der in Kunststoffolien eingeschweißt ist, sind beispielsweise schon aus der DE 90 10 426.9 bekannt.

Derartige Brustprothesen werden aus kosmetischen Gründen nach krebsbedingten Brustamputationen aus kosmetischen Gründen eingesetzt. In jüngster Zeit setzen sich neue Operationsmethoden durch, bei welchen nur noch die krebsbefallenen Teile der Brust operiert werden, hier also nur noch eine Teilamputation vorgenommen wird. Hierdurch bedingt entstehen individuell unterschiedlich unregelmäßige Narbenbereiche, die - ebenso wie das stehengebliebene Restgewebe - als solche undefinierte Oberflächen bilden. Beim Tragen herkömmlicher Brustprothesen entsteht nun durch diese unregelmäßige Oberfläche das Problem, daß häufig der weich-elastisch eingestellte Kunststoff aufgrund seiner Eigenelastizität den Narbenbereich der nur teilamputierten Brust beaufschlagt und dadurch zu einer Verringerung des Tragekomforts führt. Bei entsprechend großem Restgewebebestand paßt die Brustprothese häufig nicht mehr.

Aus der DE 44 21 516 A ist eine gattungsgemäße Zwei-Kammer-Brustprothese bekannt, bei der die zweite Kammer mit einem Polsterkörper aus Schaumkunststoff oder einem Faserknäuel gefüllt ist.

Aufgabe der vorliegenden Erfindung ist es, eine Brustprothese an die Hand zu geben, die sich insbesondere auch zur Versorgung von Patientinnen nach einer Teilamputation einer Brust eignet.

Erfindungsgemäß wird diese Aufgabe ausgehend von einer gattungsgemäßen Brustprothese dadurch gelöst, daß das Füllmaterial der zweiten Kammer aus einem vernetzenden Material besteht, das sich an die unebene Narbensituation der teilamputierten Brust beim Anlegen der Brustprothese anpaßt und diese Form im wesentlichen beibehält.

Besonders vorteilhaft ist die Verwendung eines aushärtbaren Materials. Bevorzugt verwendete Materialien sind beispielsweise eine additionsvernetzende Zwei-Komponenten Silikon-Kautschuk-Masse, wobei die Aushärtung mittels eines Platin-Katalysators sowie eines Vernetzungsmittels erfolgt, vorgemischter additionsvernetzender Zwei-Komponenten Silikon-Kautschuk, wobei die Aushärtung mittels UV-Licht erfolgt, Zwei-Komponenten Polyurethan-Gießmasse (Polyurethan-Gießmasse), sowie Zwei-Komponenten Polyurethan-Schaum.

Eine solche Brustprothese wird individuell angepaßt, indem die zweite Kammer direkt an der Patientin oder nach einem vorliegenden Abdruck des Narbenbereichs mit vernetzendem bzw. aushärtbarem Material ganz oder teilweise gefüllt wird.

Bevorzugt ist die Vernetzung innerhalb eines kurzen Zeitraums im wesentlichen abgeschlossen, wobei es zu nur sehr geringer, bevorzugt keiner, Wärmeentwicklung oder Entwicklung von Spaltmolekülen kommt. Dabei können der Mischung Leichtfüllstoffe, vorzugsweise Kunststoff-Microspheres, beigemengt sein.

Eine besondere Ausgestaltung der Erfindung liegt darin, daß die ebenfalls aus einer Kunststoffolie bestehende Außenwand der zweiten Kammer ein Füllventil aufweist, über welches das Füllmaterial einfüllbar ist. Hierdurch kann individuell durch ein eventuelles Teilbefüllen oder ein entsprechend angepaßtes Befüllen der zweiten Kammer ein entsprechender Zwischenraum geschaffen werden, der eine bessere Anpassung der Brustprothese an die Operationsnarbe schafft.

Die schalenförmige erste Kammer kann mit ihrem Randbereich über den Rand der zweiten Kammer hinausragen, so daß sich eine Höhlung ergibt. Andererseits kann der Rand der zweiten Kammer auf dem Rand der ersten Kammer aufliegen, so daß sich nur eine im Schnitt leicht konkave Wölbung ergibt.

Bei Bedarf kann die Brustprothese als Teilprothese ausgeführt sein, die nur dem zu ersetzenden Brustteil entspricht.

Weitere Einzelheiten und Vorteile der Erfindung werden nachstehend anhand einem in der Zeichnung dargestellten Ausführungsbeispiel näher erläutert. In dieser zeigen:
- Fig.1:: eine schematische Schnittdarstellung einer ersten Ausführungsform der erfindungsgemäßen Brustprothese,
- Fig. 2:: einen Schnitt durch eine zweite Ausführungsform einer erfindungsgemäßen Brustprothese,
- Fig.3:: eine Rückansicht der Ausführungsform gemäß Fig.2 vollständig gefüllter zweiter Kammer und
- Fig.4:: eine Rückansicht wie in Fig.3, jedoch mit teilbefüllter zweiter Kammer und
die
- Fig.5 und 6:: jeweils Rückansichten ähnlich der Fig. 3 und 4 mit verschiedenen Füllvorrichtungen.

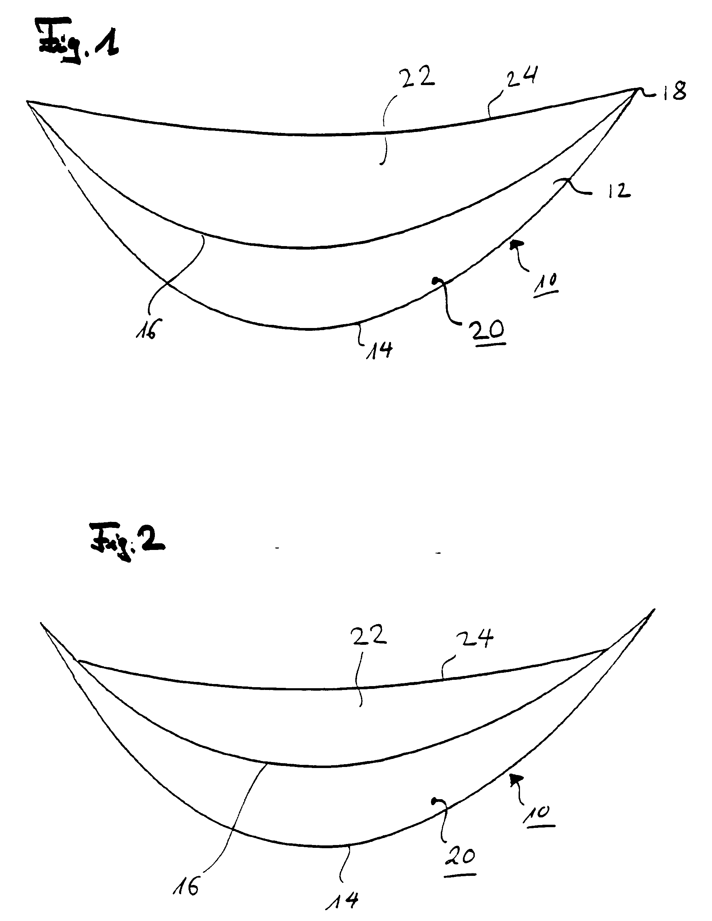

Die in Fig.1 dargestellte Brustprothese 10 besteht aus einem schalenförmigen Körper 12 aus einer weich-elastisch eingestellten additionsvernetzten Zwei-Komponenten-Silikon-Kautschuk-Masse, dessen Außenseite durch eine Polyurethanfolie 14 und dessen Innenseite durch eine Polyurethanfolie 16 abgedeckt sind, die längs ihres gemeinsamen Umfangrandes 18 durch eine umlaufende Schweißnaht miteinander verschweißt sind. Hierdurch entsteht eine erste Kammer 20. Innerhalb der durch die schalenförmige Ausbildung der ersten Kammer 20 bedingten Höhlung ist eine zweite Kammer 22 angeordnet, die durch eine ebenfalls als Polyurethanfolie ausgebildete Wandung 24 begrenzt ist. Die Polyurethanwandungen 14, 16 und 24 weisen in dem Ausführungsbeispiel gemäß Fig.1 vorteilhaft einen gemeinsamen Umfangsrand 18, der als gemeinsame Schweißnaht ausgebildet ist, auf. In der zweiten Kammer 22 ist erfindungsgemäß ein vernetzendes Material eingefüllt, welches bevorzugt im wesentlichen auch aushärtbar ist. Bevorzugt verwendete Materialien sind hierbei additionsvernetzender Zwei-Komponenten Silikon-Kautschuk, wobei dessen Aushärtung mittels eines Platin-Katalysators sowie einem Vernetzungsmittel erfolgt, ein vorgemischter Zwei-Komponenten additionsvernetzender SI-Kautschuk, wobei dessen Aushärtung mittels UV-Licht erfolgt, eine Zwei-Komponenten Polyurethan-Gießmasse, sowie ein Zwei-Komponenten Polyurethan-Schaum. Es können ferner in diesen Materialien Kunststoff-Microspheres vorgesehen sein. Die Kunststoff-Microspheres dienen dazu, das spezifische Gewicht der Gesamtmischung zu verringern und die gesamte Brustprothese dadurch leichter zu machen.

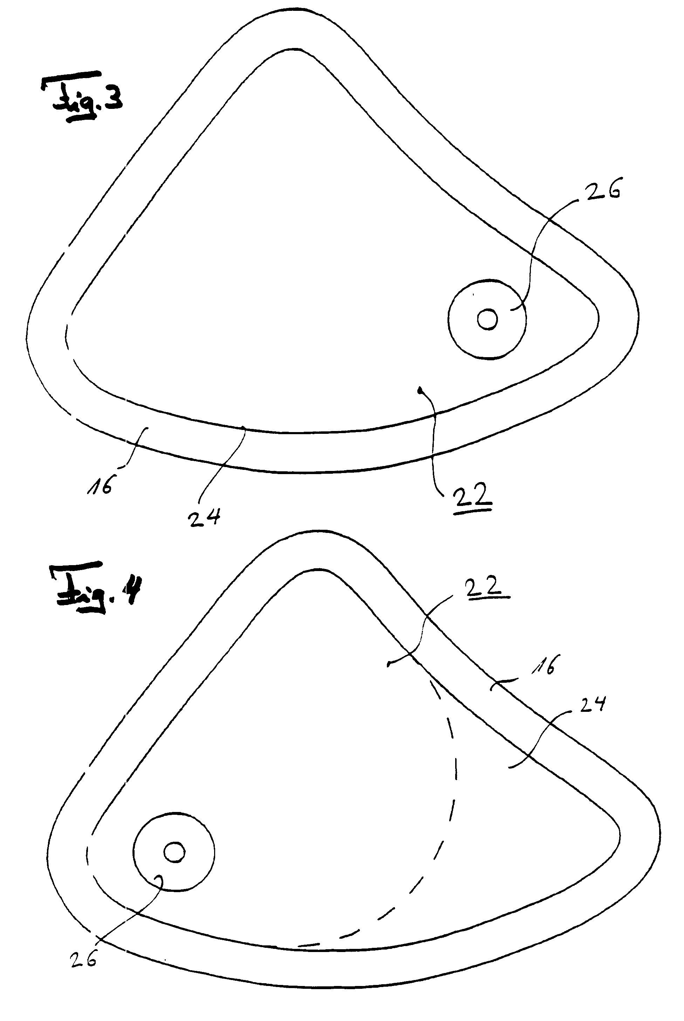

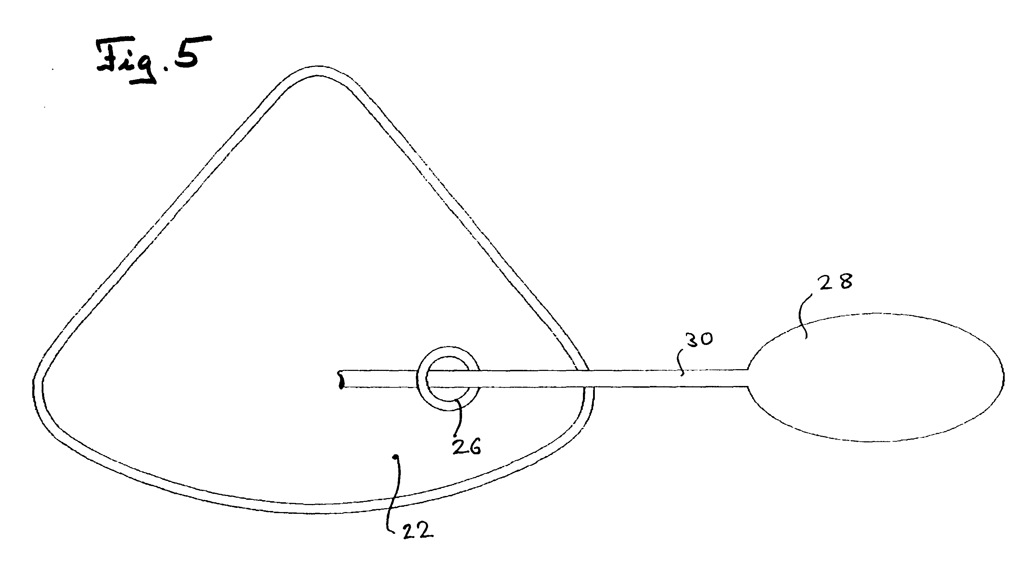

In Fig.2 ist eine abgeänderte Form einer erfindungsgemäßen Brustprothese 10 gezeigt, bei der der Randbereich der ersten Kammer über den Rand der zweiten Kammer hinausragt. Hier wird also eine flache Höhlung gebildet, wie sie sich aus dem Schnitt in Fig.2 ergibt. In der Rückansicht gemäß Fig.3 wird deutlich, daß in der Kunststoffolie 24 ein Einfüllventil 26 integriert ist. Über dieses Einfüllventil kann das vernetzende Material in die zweite Kammer 22 eingefüllt werden. Dabei kann über die eingefüllte Menge eine optimale Anpassung der Oberfläche der zweiten Kammer an den Narbenbereich der teilamputierten zweiten Brust vorgesehen werden. Vorteilhaft kann beispielsweise eine Brustprothese mit gar nicht oder nur teilbefüllter zweiter Kammer in den Handel abgegeben werden, wobei dort die Prothese durch entsprechendes Auffüllen der zusätzlichen Kammer mit dem vernetzenden Material an die aktuelle Narbensituation der Trägerin angepaßt werden kann. Durch das erfindungsgemäße Füllmaterial für die zweite Kammer ist es nun gewährleistet, daß sich diese beim Auffüllen mit dem vernetzenden Material an die Narbensituation anpaßt und in dieser Stellung formstabil verbleibt. In der Darstellung gemäß Fig.4 ist eine nur teilbefüllte zweite Kammer gezeigt, wobei über die gestrichelte Linie der Grad der Teilbefüllung angedeutet ist.

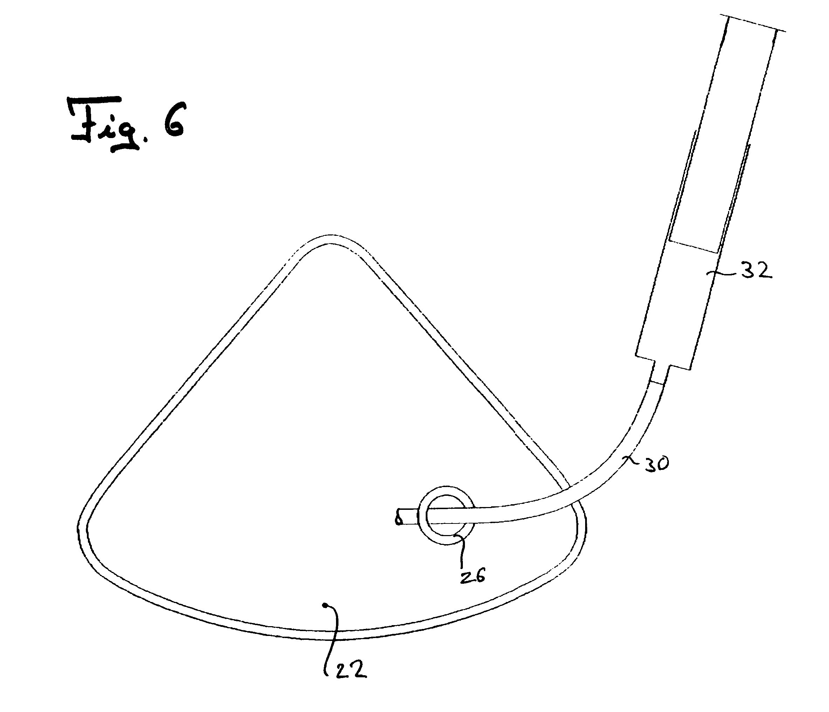

In den Figuren 5 und 6 ist jeweils in einer Rückansicht eine Brustprothese gezeigt, bei der gerade die zweite Kammer 22 über ein entsprechendes Füllsystem mit dem vernetzenden Material befüllt wird. Bei Fig. 5 besteht das Füllsystem aus einem kompressiblen Beutel 28, der das zu vernetzende Material enthält und von dem aus dieses Material über einen Schlauch 30, der über ein Ventil 26 in die zweite Kammer 22 eingeführt ist, entleert wird. Diese kompressiblen Beutel 28 können mit dem zu vernetzenden Material, das beispielsweise durch UV-Licht aushärtbar ist, befüllt verkauft werden. Dabei kann beispielsweise eine Brustprothese mit leerer zweiter Kammer und ein entsprechend gefülltes Füllsystem 28 mit Schlauch 30 als anpaßbares Brustprothesenfett eine Verkaufseinheit bilden.

In Fig. 6 ist eine alternative Ausführungsform für eine Füllvorrichtung dargestellt. Hier ist eine übliche Spritze 32 dargestellt, an die ebenfalls ein Schlauch 30 angeschlossen ist. Der Schlauch 30 ist, wie schon anhand des Beispiels von Fig. 5 dargestellt, über das Ventil 26 in die zweite Kammer 22 eingeführt.

## Patentansprüche

1. Brustprothese (10), bestehend aus einem schalenförmigen Körper (12) aus einem weich-elastisch eingestellten Kunststoff, vorzugsweise einer additionsvernetzten Zwei-Komponenten-Silikon-Kautschuk-Masse, der in Kunststoffolien (14, 16, 24) eingeschweißt ist und so eine erste Kammer (20) bildet, und einer der Trägerin während des Tragens zugewandten und sich an die erste Kammer (20) anschließenden mit einem anderen Füllmaterial gefüllten zweiten Kammer (22),
**dadurch gekennzeichnet,**
daß das Füllmaterial der zweiten Kammer (22) aus einem vernetzenden Material besteht.

2. Brustprothese (10) nach Anspruch 1, dadurch gekennzeichnet, daß das vernetzende Material aushärtbar ist.

3. Brustprothese (10) nach Anspruch 2, dadurch gekennzeichnet, daß das Material im wesentlichen eine additionsvernetzende Zwei-Komponenten-Silikon-Kautschuk-Masse ist, wobei deren Aushärtung mittels eines Platin-Katalysators sowie eines Vernetzungsmittels erfolgt.

4. Brustprothese (10) nach einem der Ansprüche 2, dadurch gekennzeichnet, daß das Material im wesentlichen aus einer additionsvernetzenden vorgemischten Zwei-Komponenten Silikon-Kautschuk-Masse besteht, deren Aushärtung mittels UV-Licht erfolgt.

5. Brustprothese (10) nach Anspruch 1 oder 2, dadurch gekennzeichnet, daß das Material im wesentlichen aus einer Zwei-Komponenten Polyurethan-Gießmasse besteht.

6. Brustprothese (10) nach Anspruch 1 oder 2, dadurch gekennzeichnet, daß das Material im wesentlichen aus einem Zwei-Komponenten Polyurethan-Schaum besteht.

7. Brustprothese (10) nach einem der vorstehenden Ansprüche, dadurch gekennzeichnet, daß dem verwendeten Material Leichtfüllstoffe, vorzugsweise Kunststoff-Microspheres, beigemengt sind.

8. Brustprothese (10) nach einem der vorstehenden Ansprüche, dadurch gekennzeichnet, daß das Füllmaterial für die zweite Kammer (22) im wesentlichen ohne Wärmeentwicklung sowie im wesentlichen ohne Entwicklung von Spaltmolekülen vernetzbar ist.

9. Brustprothese (10) nach einem der vorstehenden Ansprüche, dadurch gekennzeichnet, daß die ebenfalls aus einer Kunststoffolie bestehende Außenwand der zweiten Kammer (22) ein Füllventil (26) aufweist, über welches das Füllmaterial einfüllbar ist.

10. Brustprothese (10) nach einem der vorstehenden Ansprüche, dadurch gekennzeichnet, daß die schalenförmige erste Kammer (20) mit ihrem Randbereich über den Rand der zweiten Kammer (22) hinausragt.

11. Brustprothese (10) nach einem der vorstehenden Ansprüche, dadurch gekennzeichnet, daß der Rand der zweiten Kammer (22) auf dem Rand der ersten Kammer liegt.

12. Brustprothese (10) nach einem der vorstehenden Ansprüche, dadurch gekennzeichnet, daß sie als Teilprothese ausgeführt ist.

13. Verfahren zur Anpassung einer Brustprothese (10) nach einem der vorstehenden Ansprüche, wobei die zweite Kammer (22) gemäß einem vorliegenden Abdruck des Brustbereichs einer Patientin ganz oder teilweise mit vernetzendem Material gefüllt wird.

## Claims

1. Mammary prosthesis (10) consisting of a shell-like body (12) of a plastic with flexible elastic properties, preferably an addition-crosslinked two-component silicone rubber compound, which is welded into plastic sheets (14, 16, 24) so forming a first chamber (20), and a second chamber (22) turned towards the bearer while being worn, that is connected to the first chamber (20) and filled with another filler material,
**characterised in that**
the filler material of the second chamber (22) consists of a crosslinking material.

2. Mammary prosthesis (10) according to Claim 1, characterised in that the crosslinking material is curable.

3. Mammary prosthesis (10) according to Claim 2, characterised in that the material is essentially an addition-crosslinked two-component silicone rubber compound, the curing of which is initiated by a platinum catalyst and a crosslinking agent.

4. Mammary prosthesis (10) according to Claim 2 or 3, characterised in that the material essentially consists of an addition crosslinked pre-mixed two-component silicone rubber compound, the curing of which is initiated by UV light.

5. Mammary prosthesis (10) according to Claim 1 or 2, characterised in that the material essentially consists of a two-component polyurethane casting compound.

6. Mammary prosthesis (10) according to Claim 1 or 2, characterised in that the material essentially consists of a two-component polyurethane foam.

7. Mammary prosthesis (10) according to one of the preceding Claims, characterised in that light filler materials, preferably plastic microspheres, are added to the material used.

8. Mammary prosthesis (10) according to one of the preceding Claims, characterised in that the filler material for the second chamber (22) is essentially crosslinkable without heat evolution and also essentially without the development of decomposition molecules.

9. Mammary prosthesis (10) according to one of the preceding Claims, characterised in that the outer wall of the second chamber (22), likewise consisting of a plastic sheet material, exhibits an inlet valve (26) via which the filler material can be introduced.

10. Mammary prosthesis (10) according to one of the preceding Claims, characterised in that the edge zone of the shell-shaped first chamber (20) extends beyond the edge of the second chamber (22).

11. Mammary prosthesis (10) according to one of the preceding Claims, characterised in that the edge of the second chamber (22) lies on the edge of the first chamber.

12. Mammary prosthesis (10) according to one of the preceding Claims, characterised in that it is designed as a partial prosthesis.

13. Process for adapting a mammary prosthesis (10) according to one of the preceding Claims, in which the second chamber (22) is completely or partially filled with crosslinking material in accordance with a prepared impression of the breast region of a patient.

## Revendications

1. Prothèse mammaire (10) consistant en un corps en forme de coque (12) en un matériau plastique présentant des propriétés molles élastiques, de préférence une masse à deux composants silicone/caoutchouc réticulée par addition, qui est soudé dans des films en plastique (14, 16, 24) et qui forme ainsi une première chambre (20), et une seconde chambre (22) dirigée vers la porteuse pendant qu'elle porte la prothèse et qui est contiguë à la première chambre (20) et est remplie d'un autre matériel de remplissage,
caractérisée en ce que le matériel de remplissage de la seconde chambre (22) consiste en un matériau réticulant.

2. Prothèse mammaire (10) selon la revendication 1, caractérisée en ce que le matériau réticulant est durcissable.

3. Prothèse mammaire (10) selon la revendication 2, caractérisée en ce que le matériau consiste essentiellement en une masse à deux composants silicone/caoutchouc réticulant par addition, son durcissement se faisant moyennant un catalyseur de platine ainsi qu'un moyen de réticulation.

4. Prothèse mammaire (10) selon les revendications 2 ou 3, caractérisée en ce que le matériau consiste essentiellement en une masse à deux composants silicone/caoutchouc prémélangée et réticulant par addition dont le durcissement se fait par lumière UV.

5. Prothèse mammaire (10) selon les revendications 1 ou 2, caractérisée en ce que le matériau consiste essentiellement en une masse à deux composants en polyuréthane coulé.

6. Prothèse mammaire (10) selon les revendications 1 ou 2, caractérisée en ce que le matériau consiste essentiellement en une mousse de polyuréthane à deux composants.

7. Prothèse mammaire (10) selon l'une quelconque des revendications précédentes, caractérisée en ce qu'au matériau utilisé des matières de remplissage légères sont ajoutées, de préférence des microsphères en plastique.

8. Prothèse mammaire (10) selon l'une quelconque des revendications précédentes, caractérisée en ce que le matériau de remplissage pour la seconde chambre (22) peut être réticulé essentiellement sans développer de la chaleur ainsi qu'essentiellement sans le développement de molécules de fission.

9. Prothèse mammaire (10) selon l'une quelconque des revendications précédentes, caractérisée en ce que la paroi extérieure de la seconde chambre (22) consistant également en un film plastique présente une soupape de remplissage (26) par laquelle le matériel de remplissage peut être rempli.

10. Prothèse mammaire (10) selon l'une quelconque des revendications précédentes, caractérisée en ce que la première chambre (20) en forme de coque dépasse par sa zone de bordure le bord de la seconde chambre (22).

11. Prothèse mammaire (10) selon l'une quelconque des revendications précédentes, caractérisée en ce que le bord de la seconde chambre (22) repose sur le bord de la première chambre.

12. Prothèse mammaire (10) selon l'une quelconque des revendications précédentes, caractérisée en ce qu'elle est exécutée comme prothèse partielle.

13. Procédé pour l'adaptation d'une prothèse mammaire (10) selon l'une quelconque des revendications précédentes, la seconde chambre (22) étant entièrement ou partiellement remplie d'un matériau réticulant selon une empreinte faite de la zone du sein d'une patiente.
